# EUROPEAN PATENT APPLICATION

(11) **EP 2 341 343 A1**
(43) Date of publication of application: **06.07.2011**
(21) Application number: 10195534.2
(22) Date of filing: 17.12.2010
(51) Int. Cl.: G01N 33/487, G06K 19/077

(54) **Measurement system and method for determination and/or detection of one or more agents in one or more samples using said measurement system**

(30) Priority: 18.12.2009 EP 09180021
(71) Applicant: Virco BVBA, 2340 Beerse (BE); Ortho Clinical Diagnostics Inc., Rochester, New York 14626-5101 (US)
(72) Inventor: Öhman, Ove, 75591 Uppsala (SE); Stuyver, Lieven Jozef, 9552 Herzele (BE)
(74) Representative: Daelemans, Frank F.R.

(57) **Abstract**

The present invention relates to a measurement system which comprises a reader and a chip and concerns a method to determining and detecting biological and chemical agents or materials in biological samples.

## Description

The present invention relates to a measurement system which comprises a reader and a chip and concerns a method to determining and detecting biological and chemical agents or materials in biological samples.

As pharmaceutical science, knowledge of the human genome and technology as such advance, the increasing understanding of human disease brings scientific results to new frontiers for clinical application. For instance, a better understanding of the mechanism underpinning specific pathologies results in the identification of biomarkers, specific biological traits that can be used to measure the progress of a disease or treatment.

Biomarkers that are up or down regulated in an illness or wellness of a patient should be measured and that by measuring those biomarkers patients can be targeted for efficient pharmaceutical treatment. Therefore it is necessary to measure these biomarkers and tailor to the right treatment.

Some of the research, development, and medical product review processes are focused on more effective, targeted therapies. This knowledge is being translated into clinical tests to monitor drug therapy, thereby enabling health care providers to select drugs that work safely for specific patients, patient groups and/or conditions. To ensure widespread adoption of this knowledge the medical or genetic test information must be both clinically and analytically valid. Pharmacogenomics, metabolomics, proteomix, transcriptomics or the identification of genes that relate to drug treatment enables personalized health care with treatment and prevention tailored to each person's needs.

Advances in technology, biomedical research, and medicine often take many years to be adopted throughout the health care delivery system. The rapid rate of scientific and medical advances outstrips the ability of clinicians and providers to remain up-to-date on the latest medical information. Better and more efficient ways to provide useful information to support clinical decisions of health care providers and consumers are definitively needed. The lack of user-friendly information sources often hampers adoption of newer approaches, such as the incorporation of genetic testing practices in routine clinical decision-making.

To support the readiness of health care professionals and change clinical practice patterns to adopt best practices, robust clinical decision support and information management tools will need to be integrated into electronic health records and other health information technology systems. New provider education in the use of genomic information is also likely to be needed.

The translation of advances into health care delivery and the adoption of best practices require strong medical evidence. Personalized health care must be strongly aligned with the development and use of evidence-based care. The adoption of new tests, therapies, and techniques will be strongly affected by evidence of their clinical and economic value. The development of medical evidence focused on patient outcomes, and the ability to compare alternatives, will be an increasingly important element of health care. The development of easy to use and reliable systems for generating evidence at the point of health care delivery can add significantly to the evidence base and to growing knowledge of individual variations in response to treatments.

Biosensors, for instance, have long been an important part of health care in hospitals and some managed care facilities.

A biosensor is a device for the detection of an analyte or agent that combines a biological component with a physicochemical detector component.

It consists of 3 parts:
- The *probe* or sensitive biological element: A biological material such as tissue, microorganisms, organelles, cell receptors, enzymes, antibodies, nucleic acids, or other biologically derived materials. The sensitive element can be created by biological engineering.
- the *transducer* or the *detector element* that transforms the signal resulting from the interaction of the analyte or agent with the biological element into another signal (i.e., transduces) that can be more easily measured and quantified;
- a *signal processor,* or electronic component that is primarily responsible for the capture of the signal and the delivery of the results in a user-friendly way.

Many technologies have been proposed for biosensors that can be used at home, including disposable or single-use devices. Biosensors for personal use at home or, more generally, outside of hospitals or medical clinics, offer many opportunities for improved health care.

The most widespread example of a commercial biosensor is the blood glucose biosensor, which uses the enzyme glucose oxidase to break blood glucose down.

In the prior art measurement systems and methods are known for detecting biological and/or chemical material, wherein the measurement system comprises a reaction cell for containing a measurement apparatus and an external control unit.

The measurement apparatus comprise probes, transducers, and a signal processor that includes an information communication device and an information storage device for storing information. The external control unit and information communication device of the measurement apparatus exchange information, without having physical contact with one another.

Recent technological advances have permitted the miniaturization of the measurement apparatus to the size of a small chip, which enables it to be conveniently integrated in an easily portable device, or even implanted in the body of a patient. However, in prior art the energy needed for the proper functioning of the measurement apparatus is obtained from a device in which this energy is stored beforehand and therefore finite. To maintain the portability or implantability of the measurement system, this energy storage device is also required to be small. However, the amount of energy that can be stored in a conveniently small device, e.g. a chemical battery, is frequently too limited to provide sufficient power to the measurement apparatus to allow it to function properly over a long period of time. The energy storage device therefore needs to be recharged or replaced on a regular basis.

The size of the antenna will define the size of the chip, and hence also the cost price. It is not the size of the biosensor head that is driving the size of the chips. Small chips that are floating in the liquid are so small that the antenna will not capture enough energy from RFID. Therefore additional energy sources need to be applied.

It is therefore an object of this invention to provide a new, alternative measurement system not having these disadvantages. Accordingly the current invention relates to a
system comprising of a reader and a chip, for instance a disposable chip, said chip comprises
a) a specific probe and a transducer connected to said probe,
b) a signal processor including one or more components that support the storage and communication of information and
c) an energy harvesting component.

In addition to the inventive measurement system above described, an external control unit is configured to perform information communication by either sending or receiving a signal from said signal processor, and the energy for functioning of the chip is delivered by said energy harvesting component.

The specific probe is biological material such as an antigen, antibody, nucleic acid, peptide or protein while the signal processor is an active radio frequency identification tag.

The sending or receiving of the signal from the signal processor occurs preferably via wireless transmission.

In addition to the foregoing the energy harvesting component obtains its energy by collecting said energy from an external source such as a solar power source, a light or laser source, a heat source, a magnetic source, salinity gradients and / or kinetic energy.

Furthermore the invention concerns a method of determination or detection of one or more agents in one or more samples, the method comprising:
depositing the chip as defined above in a biological sample,
activating the chip through the energy harvesting component whereby said energy harvesting component collects its energy from an external source before, after or at the moment when the chip is brought in contact with said biological sample,
allowing the chip for a period of time in contact with said biological sample in order to react the agent with the specific probe and transducer connected to said probe
where after or as a result of said reaction the signal processor transmits a signal to the external control unit on which information is displayed in relation to the determination or detection of one or more agents in said one or more samples.
In the above chip, the specific probe and transducer connected to said probe can be composed of one of the following:
- Metal-oxide semiconductor field effect transistor (mosfet) to amplify or switch electronic signals,
- a chemical field-effect transistor (chemfet) using a chemical sensor that transduces the chemical signal in an action potential;
- a ion-sensitive field-effect transistor (isfet) using a chemical sensor to detect ions in electrolytes;
- a enzyme field effect transistor (enfet) using a sensor specialized for detection of specific enzymen converting the substrate thereby generating H+ or OH- which can be detected by isfet
- an electrolyte-insulator-semiconductor (eisfet) using a sensor to measure the electrolyte
- a biosensor coupled with nucleic acid probes that can detect corresponding nucleic acids in the direct environment of the biosensor
- a biosensor coupled with antigens that can detect specific antibodies in the direct environment of the biosensor
- a biosensor coupled with antibodies that can detect specific antigens in the direct environment of the biosensor
- a biosensor coupled with nucleic acids that can detect specific nucleic acid binding proteins in the direct environment of the biosensor
- a light-addressable potentiometric sensor (LAPS) using light (e.g. LEDs) to select what will be measured
- or any other arrangement measuring a molecular interaction through an electrical or optical transducer

In the method described above the specific probe may be a biological material such as an antigen, antibody, nucleic acid, peptide or protein while the signal processor may be an active radio frequency identification tag.

The sending or receiving of the signal from the signal processor occurs preferably via wireless transmission.

In addition to the foregoing, the energy harvesting component obtains its energy by collecting said energy from an external source such as a solar power source, a light or laser source, a heat source, a magnetic source, salinity gradients and / or kinetic energy.

Energy harvesting, as mentioned above, is also called power harvesting or energy scavenging, and is the process by which energy is derived from external sources (e.g., solar power, thermal energy, wind energy, salinity gradients and kinetic energy), captured and stored. Frequently this term is applied when speaking about small, wireless autonomous devices like those used in wearable electronics and wireless sensor networks.

The history of energy harvesting dates back to the windmill and the waterwheel. People have searched for ways to store the energy from heat and vibrations for many decades. One driving force behind the search for new energy harvesting devices is the desire to power sensor networks and mobile devices without batteries.

Energy harvesting devices converting ambient energy into electrical energy have attracted much interest in both the military and commercial sectors. Some systems convert motion into energy, for example the motion of ocean waves is converted into electricity to be used by oceanographic monitoring sensors for autonomous operation. Another application is in wearable electronics, where energy harvesting devices can power or recharge cell phones, mobile computers, radio communication equipment, etc. All of these devices must be sufficiently robust to endure long-term exposure to hostile environments and have a broad range of dynamic sensitivity to exploit the entire spectrum of motions.

Examples of energy harvesting applicable to the current invention are small scale energy sources such as:
- Piezoelectric crystals or fibers that generate a small voltage if they are mechanically deformed. Vibration from engines can stimulate piezoelectric materials.
- Some wristwatches are already powered by kinetic energy (called kinetic watches), in this case the movement of the arm. The arm movement causes a magnet in a small electromagnetic generator to move. The motion provides a rate of change of flux, which results in an induced electromagnetic force on the coils, in accordance with Faraday's Law.
- Thermo-electric generators (TEGs) consist of a junction of two dissimilar conductive materials which are in a thermal gradient. Large voltage outputs are possible by connecting many junctions electrically in series and thermally in parallel. Typical performance is 100-200 microV/degreeC per junction. These can be utilized to capture mW of energy from industrial equipment, structures, and even the human body.
- Micro wind turbines are used to harvest kinetic energy readily available in the environment in the form of wind, to power low power electronic devices such as wireless sensor nodes. When air flows across the blades of the turbine, a net pressure difference is developed above and below the blades. This will result in a lift force generated which in turn rotates the blades. This is known as the aerodynamic effect.

One idea is to deliberately broadcast RF energy to power remote devices: This is now commonplace in passive Radio Frequency Identification (RFID) systems.

Piezoelectric systems can convert motion from the human body into electrical power. Energy from leg and arm motion, shoe impacts, and blood pressure can be used to provide low level power to implantable or wearable sensors. Most piezoelectric electricity sources produce power on the order of milli-watts, too small for system application, but enough for hand-held devices. There is also a proposal to use them in micro-scale devices, for example in a device harvesting micro-hydraulic energy. In this device, the flow of pressurized hydraulic fluid drives a reciprocating piston supported by three piezoelectric elements, which convert the pressure fluctuations into an alternating current.

The pyro-electric effect as well as the Seebeck effect converts a temperature change into electrical current or voltage. It is analogous to the piezoelectric effect, but the electric field is created by heating the material instead of by applying a mechanical strainMiniature thermocouples have been developed that convert body heat into electricity and generate 40 microW at 3V with a 5 degree temperature gradient while on the other end of the scale, large thermocouples are used in nuclear RTG batteries. Practical examples are the finger-heart rate meter and the thermo-generators.

Advantages to thermo-electrics:
1. The absence of moving parts allows continuous operation for many years.
2. Thermo-electrics contain no materials that must be replenished.
3. Heating and cooling can be reversed.

One downside to thermoelectric energy conversion is low efficiency (currently less than 10%). The development of materials that are able to operate in higher temperature gradients, and that can conduct electricity well without also conducting heat (something that was until recently thought impossible), will result in increased efficiency.

Another way of energy harvesting is through the oxidation of blood sugars. These energy harvesters are called bio-fuel cells. They could be used to power implanted electronic devices (e.g., pacemakers, implanted biosensors for diabetics, implanted active RFID devices, etc.).

Electroactive polymers (EAPs) have been proposed for harvesting energy. These polymers have a large strain coefficient, high elastic energy density, and high energy conversion efficiency. The total weight of systems based on EAPs is proposed to be significantly lower than those based on piezoelectric materials.

Nanogenerators could provide a new way for powering devices without batteries, although existing prototypes generate only tens of nano-watts. As this is too low for any application, further development is needed.

Another way of energy harvesting is by galvanic power. A galvanic cell converts chemical energy into electrical energy by using spontaneous chemical reactions that take place at the electrodes. Each galvanic cell has its own characteristic voltage (defined as the energy release per electron transfer from one electrode to the other). A simple galvanic cell will consist only of an electrolyte and two different electrodes. (Galvanic cells can also be made by connecting two half cells, each with its own electrode and electrolyte, by an ion-transporting "bridge", usually a salt bridge; these cells are more complex.) The electrodes typically are two metals, which naturally have different reaction potentials relative to the electrolyte. This causes ions of one of the electrodes to preferentially enter the solution at one electrode, and another ion to leave the solution at the other electrode. This generates an electric current across the electrolyte, which will drive electric current through a wire that makes an exterior connection to each of the electrodes. A galvanic cell uses electrodes of different metals, whereas an electrolytic cell may use the same metal for cathode and anode.

Some embodiments of the measurement system of the current invention comprising the chip are for instance:
a. A device of hourglass shape, in which a sample caused to pass over the chip by gravity or by a pressure differential.
   The basic idea is to let the sample flow from one compartment to another compartment and in the middle let it passes a smaller conduct where the detection is being performed. This means that the sender/receiver/energizer could be more precise in addressing the RFID device. The arrangement could be visualized as an hourglass. The flow could be passively or actively enabled.
b. A device based on capillary action.
   Finger pricks are often performed with glass or polymer capillaries. If the chip is incorporated in the capillary system, a good method of bringing the fluid in contact with the chip can be combined with a good system for sampling.
c. A system that uses magnets to bring the chip to a well-defined position for is placed for reading and facilitating energy transfer, and optionally also to enhance reactions by agitation.
   By depositing a paramagnetic layer on the chip, the chip can be treated as a magnetic bead. Magnets can then be used to bring the chip to a desired position, which may be advantageous for sampling a specific area, for transferring energy to it in a more controlled fashion, and for receiving signal from the device more efficiently. Magnetic manipulation may also be used to enhance the mixing of fluids. Optionally, this technique could be combined with a device based on capillary flow.
d. A device for in-vivo physical measurements of, for instance, blood pressure and/or blood flow, in the human body and in specific organs.
   Many sensors other than those performing chemical or biological detection can be integrated into the same chip. Examples are pressure sensors and possibly flow speed sensors. Integrating such sensors into a stent would be an elegant manner of keeping track of re-stenosis, and/or monitoring arterial stiffness or other (mal) functions.

## Claims

1. A measurement system comprising of a reader and a chip, said chip comprises
a) a specific probe and a transducer connected to said probe,
b) a signal processor including one or more components that support the storage and communication of information, and
c) an energy harvesting component.

2. A measurement system according to claim 1 further comprising an external control unit which is configured to communicate by sending and/or receiving a signal from said signal processor, and the energy for functioning of said chip is delivered by said energy harvesting component.

3. A measurement system according to claim 1 or 2 wherein said specific probe comprises biological material such as an antigen, antibody, nucleic acid, peptide or protein.

4. A measurement system according to claims 1 - 3 wherein the signal processor is an active radio frequency identification tag.

5. A measurement system according to claims 1-4 wherein said sending or receiving the signal from the signal processor occurs via wireless transmission.

6. A measurement system according to any of the preceding claims wherein the energy harvesting component obtains its energy by collecting said energy from an external source such as a solar power source, a light or laser source, a heat source, a magnetic source, salinity gradients and / or kinetic energy.

7. A method of determination or detection of one or more agents in one or more samples, the method comprising:
depositing the chip as defined in any of the claims 1-6 in a biological sample,
activating the chip through the energy harvesting component whereby said energy harvesting component collects its energy from an external source before, after or at the moment at which the chip is brought into contact with said biological sample,
allowing the chip to remain in contact with said biological sample for a period of time in order to react the agent with the specific probe and transducer connected to said probe
where after or as a result of said reaction the signal processor transmits a signal to the external control unit, on which information is displayed in relation to the determination or detection of one or more agents in said one or more samples.

8. A method according to claim 7 wherein the specific probe is biological material such as an antigen, antibody, nucleic acid, peptide or protein;

9. A method according to claim 7 or 8 wherein the signal processor is an active radio frequency identification tag.

10. A method according to claims 7-9 wherein the transmission of the signal from the signal processor to the external control unit occurs via wireless transmission.

11. A method according to any of the claims 7-10 wherein the energy harvesting component obtains its energy by collecting said energy from an external source such as a solar power source, a light or laser source, a heat source, a magnetic source, salinity gradients and / or kinetic energy.
